# EUROPÄISCHE PATENTSCHRIFT

(11) **EP 2 215 241 B1**
(45) Veröffentlichungstag und Bekanntmachung des Hinweises auf die Patenterteilung: **16.03.2011**
(21) Anmeldenummer: 08857658.2
(22) Anmeldetag: 13.11.2008
(51) Int. Cl.: C12P 5/02

(54) **VERFAHREN ZUR HERSTELLUNG VON BIOGAS UNTER VERWENDUNG EINES GÄRSUBSTRATS MIT EINGESTELLTER AMMONIUM-STICKSTOFFKONZENTRATION**
PROCESS FOR THE PRODUCTION OF BIOGAS EMPLOYING A FERMENTATION SUBSTRATE WITH ADJUSTED AMMONIUM NITROGEN CONCENTRATION
PROCÉDÉ DE PRODUCTION DE BIOGAZ UTILISANT UN SUBSTRAT DE FERMENTATION À CONCENTRATION D'AZOTE AMMONIUM AJUSTÉE

(30) Priorität: 07.12.2007 DE 102007059084
(43) Veröffentlichungstag der Anmeldung: 11.08.2010
(73) Patentinhaber: Rückert, Claus, 91207 Lauf/Pegnitz (DE)
(72) Erfinder: Rückert, Claus, 91207 Lauf/Pegnitz (DE)
(74) Vertreter: Gassner, Wolfgang
(86) Internationale Anmeldenummer: PCT/EP2008/065490
(87) Internationale Veröffentlichungsnummer: WO 2009/071428

(56) Entgegenhaltungen:
- DE-A1-102004 030 482
- DE-A1-102005 047 719
- GELEGENIS, J. ET AL.: "Optimization of biogas production by co-digesting whey with diluted poultry manure" RENEWABLE ENERGY, Bd. 32, Nr. 13, 30. März 2007 (2007-03-30), Seiten 2147-2160, XP022006422
- GELEGENIS, J. ET AL.: "Optimization of biogas production from olive-oil mill wastewater, by codigesting with diluted poultry-manure" APPLIED ENERGY, Bd. 84, Nr. 6, 6. April 2007 (2007-04-06), Seiten 646-663, XP022023910
- CALLI, B. ET AL.: "Effects of high free ammonia concentrations on the performances of anaerobic bioreactors" PROCESS BIOCHEMISTRY, Bd. 40, Nr. 3-4, 1. März 2005 (2005-03-01), Seiten 1285-1292, XP025306366
- DATABASE WPI Week 200351 Thomson Scientific, London, GB; AN 2003-536020 XP002532704 & JP 2003 039036 A (ISHIKAWAJIMA HARIMA HEAVY IND) 12. Februar 2003 (2003-02-12)

## Beschreibung

Die Erfindung betrifft ein Verfahren zur Erzeugung von Biogas aus Substraten mit einer hohen ersten Ammonium-Stickstoffkonzentration, insbesondere Geflügelkot.

Die DE 10 2005 047 719 A1 offenbart ein Verfahren zur Erzeugung von Biogas aus Biomasse mit hohen Feststoff- und Stickstoffanteilen. Dabei wird ein beim Vergären gebildeter Gärrest in zwei Teilströme getrennt. Ein erster Teilstrom wird zum Anmaischen neuer Biomasse rezirkuliert. Einem zweiten Teilstrom wird in einem Strippprozess Ammoniak/Ammoniumstickstoff entzogen und anschließend in eine feste und eine flüssige Phase getrennt. Die flüssige Phase wird ebenfalls zum Anmaischen neuer Biomasse rezirkuliert.

Beim Strippprozess wird der Ammoniumstickstoffgehalt um 70% bis 90% verringert. Infolge dessen enthält die rezirkulierte flüssige Phase des zweiten Teilstroms bis zu 30% Ammoniumstickstoff. Um einen zu hohen Gehalt an Ammonium im Gärsubstrat und eine dadurch bedingte Stickstoffhemmung zu vermeiden, kann bei dem bekannten Verfahren nur ein relativ geringer Anteil an eine hohe Ammonium-Stickstoffkonzentration aufweisenden Geflügelkot dem Gärsubstrat zugefügt werden. In der Regel beträgt der Anteil des Geflügelkots am Gärsubstrat nicht mehr als 25 Gew.-%.

Die DE 103 53 728 A1 betrifft ein verfahren zur Gewinnung von Biogas. Hier wird ein teilweise vergorenes Substrat aus dem Reaktor entnommen und in eine feste und in eine flüssige Phase getrennt. Die flüssige Phase wird durch einen Strippprozess im Wesentlichen von Ammonium und Ammoniak befreit. Die flüssige Phase wird sodann mit der festen Phase in einem Mischbehälter vermischt. 40 bis 80 Vol.-% der Fest/Flüssigmischung werden in den Reaktor zurückgeführt.

Aus der EP 1 762 607 A1 ist ein Regelungsverfahren für Biogasanlagen bekannt. Der Ammoniakwert eines Gärsubstrats wird durch eine Zudosierung entsprechender Regelsubstrate auf einen vorgegebenen Sollwert gehalten. Die Rückführung eines aufbereiteten Gärrests in den Biogasreaktor ist nicht vorgesehen.

Die DE 10 2004 030 482 A1 betrifft ein Verfahren zur Aufbereitung von Abwässern aus der Bearbeitung und Aufbereitung von organischen Abfällen, insbesondere von Gülle, mit einem Biogasfermenter. Dem Fermenter werden Gärreste entnommen und einer Fest-Flüssig-Trennung unterzogen. Ein Teil der festen Phase wird in den Fermenter rückgeführt. Die flüssige Phase wird einer Ultrafiltration und einer Umkehrosmose unterzogen. Die gereinigte flüssige Phase wird teilweise in den Fermenter rückgeführt. Ziel des Verfahrens ist die Herstellung eines einleitfähigen, insbesondere landwirtschaftlich nutzbaren, Wassers.

Das Dokument GELEGENIS, J. ET AL.: "Optimization of biogas production by co-digesting whey with diluted poultry manure" (RENEWABLE ENERGY, Bd. 32, Nr. 13, 30. März 2007, Seiten 2147-2160) offenbart ein Verfahren zur Erzeugung von Biogas aus einem Misch-Substrat, welches sich aus stickstoffreichem Geflügelmist und stickstoffarmer Molke zusammensetzt. Dabei wird gezeigt, daß der Einsatz des Misch-Substrats zu einem höheren Biogasertrag führt als der Einsatz der separaten Substrate Geflügelmist oder Molke.

Das Dokument GELEGENIS, J. ET AL.: "Optimization of biogas production from olive-oil mill wastewater, by codigesting with diluted poultry-manure" (APPLIED ENERGY, Bd. 84, Nr. 6, 6. April 2007, Seiten 646-663) offenbart ebenfalls ein Verfahren zur Erzeugung von Biogas aus einem Misch-Substrat, welches sich hier aus stickstoffreichem Geflügelmist und stickstoffarmem Oliven-Ölmühlenabwasser zusammensetzt. Es wird auch hier gezeigt, daß der Einsatz des Misch-Substrats zu einem höheren Biogasertrag führt als der Einsatz des separaten Substrats Geflügelmist.

Im Dokument CALLI, B. ET AL.: "Effects of high free ammonia concentrations on the performances of anaerobic bioreactors" (PROCESS BIOCHEMISTRY, Bd. 40, Nr. 3-4, 1. März 2005, Seiten 1285-1292) wird gezeigt, daß hohe Ammoniakkonzentrationen in anaeroben Biogasfermentern die Methanogenese inhibieren, während Konzentrationen von bis zu 5 kg Ammoniak-Stickstoff/m³ noch nicht inhibierend wirken.

Aus der DE 10 2004 003 458 A1 ist ein Verfahren zur Restmüllaufbereitung bekannt. Die Entstickung eines Prozesswassers erfolgt mittels einer Strippeinrichtung.

Derzeit steigen die Preise für nachwachsende Rohstoffe wegen steigender Nachfrage stark an. Geflügelkot ist wegen seines hohen Stickstoffgehalts dagegen in der Regel nicht ohne Weiteres zu entsorgen und wird infolge dessen kostenlos abgegeben.

Aufgabe der vorliegenden Erfindung ist es, ein möglichst einfach und kostengünstig durchführbares Verfahren zur Gewinnung von Biogas aus Gärsubstraten mit einem hohen Gehalt an Ammonium-Stickstoff, insbesondere Geflügelkot, anzugeben.

Diese Aufgabe wird durch die Merkmale des Anspruchs 1 gelöst. Zweckmäßige Ausgestaltungen ergeben sich aus den Merkmalen der Ansprüche 2 bis 21.

Nach Maßgabe der Erfindung ist ein Verfahren zur Erzeugung von Biogas aus einem Substrat mit einer ersten Ammonium-Stickstoffkonzentration von mehr als 5,0 kg NH₄-N/m³, insbesondere Geflügelkot, mit folgenden Schritten vorgesehen:
Herstellen eines eine Mischung aus dem Substrat und einem weiteren Substrat enthaltenden, Gärsubstrats,
Bestimmen einer zweiten Ammonium-Stickstoffkonzentration des Gärsubstrats und
Einstellen der zweiten Ammonium-Stickstoffkonzentration im Gärsubstrat derart, dass ein Minimalwert von 3,0 kg NH₄-N/m³ nicht unterschritten und ein Maximalwert von 5,5 kg NH₄-N/m³ nicht überschritten wird, wobei dem Gärsubstrat
   (i) zum Erniedrigen der zweiten Anunonium-Stickstoffkonzentration einem vergorenen Gärrest entzogenes und mittels eines Umkehrosmoseverfahrens gereinigtes Prozesswasser und
   (ii) zum Erhöhen der zweiten Ammonium-Stickstoffkonzentration erstes Substrat: zugesetzt wird.

Mit dem vorgeschlagenen Verfahren ist es möglich, Gärsubstrate mit einem hohen Anteil an Substraten mit einer hohen ersten Ammonium-Stickstoffkonzentration, insbesondere Geflügelkot, zu vergären. Es gelingt damit, in Abweichung vom Stand der Technik, Gärsubstrate mit einem Anteil von mehr als 30 Gew.-% an Geflügelkot in Biogas umzuwandeln. Erfindungsgemäß wird das insbesondere durch eine geeignete Einstellung der zweiten Ammonium-Stickstoffkonzentration im Gärsubstrat erreicht. Dabei wird zur besonders effizienten Verarbeitung des Substrats die zweite Ammonium-Stickstoffkonzentration vorzugsweise nahe des Maximalwerts eingestellt, beispielsweise in einem Bereich von mehr als 4,0 kg NH₄-N/m³ bis 5,5 kg NH₄-N/m³. Ein besonders bevorzugter Bereich der zweiten Ammonium-Stickstoffkonzentration reicht von einem Minimalwert von 4,5 kg NH₄-N/m³ bis zu einem Maximalwert von 5,5 kg NH₄-N/m³.

Zum Einstellen der vorgenannten Bereiche der zweiten Ammonium-Stickstoffkonzentration kann es entweder erforderlich sein, die zweite Ammonium-Stickstoffkonzentration zu erniedrigen oder zu erhöhen. Erfindungsgemäß wird zum Erniedrigen der zweiten Ammonium-Stickstoffkonzentration einem vergorenen Gärrest entzogenes und mittels eines Umkehrosmoseverfahrens gereinigtes Prozesswasser zugesetzt. Derartiges Prozesswasser weist eine äußerst geringe Ammonium-Konzentration auf. Es ist jedenfalls so sauber, dass es durch Einleiten in Vorfluter oder in Flüsse entsorgt werden kann. Indem zum Erniedrigen der zweiten Ammonium-Stickstoffkonzentration Prozesswasser verwendet wird, kann einerseits der Verbrauch von wertvollem Frischwasser vermieden und andererseits ein Eintrag von Ammonium in das Gärsubstrat vermieden werden. Infolge dessen kann dem Gärsubstrat ein besonders hoher Anteil an Substrat bzw. Geflügelkot zugesetzt werden.

Zum Einstellen des vorgenannten Bereichs der zweiten Ammonium-Stickstoffkonzentration kann es auch erforderlich sein, diese zu erhöhen. Zu diesem Zweck wird dem Gärsubstrat Substrat zugesetzt. Da das Substrat in der Regel eine erste Ammonium-Stickstoffkonzentration von mehr als 5,0 kg NH₄-N/m³, insbesondere mehr als 10,0 kg NH₄-N/m³, aufweist, kann damit die zweite Ammonium-Stickstoffkonzentration erhöht und gleichzeitig eine effiziente Entsorgung des Substrats erreicht werden.

Nach einer vorteilhaften Ausgestaltung wird zur Bestimmung der zweiten Ammonium-Stickstoffkonzentration der Ammoniumgehalt oder der Ammoniakgehalt im Gärsubstrat gemessen. Insoweit können nach dem Stand der Technik bekannte Messgeräte verwendet werden. Vorteilhafterweise wird die zweite Ammonium-Stickstoffkonzentration mittels bekannter Messgeräte laufend gemessen und überwacht.

In ähnlicher Weise kann auch die erste Ammonium-Stickstoffkonzentration im Substrat bestimmt werden. Auch zu diesem Zweck kann der Ammoniumgehalt oder der Ammoniakgehalt im Substrat gemessen werden. Dabei befindet sich das Substrat in einem gesonderten Vorratsbehälter.

Nach einer besonders vorteilhaften weiteren Ausgestaltung ist vorgesehen, dass die erste Ammonium-Stickstoffkonzentration durch Erhitzen des Substrats vor der Zugabe zum Gärsubstrat auf einen vorgegeben Wert verringert wird. Dabei kann der vorgegebene Wert in Abhängigkeit der zweiten Ammonium-Stickstoffkonzentration sowie der Menge des Gärsubstrats und der Menge des Substrats ermittelt werden. Zum Erhitzen des Substrats kann vorteilhafterweise durch Verbrennen von Biogas erzeugtes Abgas verwendet werden. Biogas kann beispielsweise zum Antrieb eines Generators verbrannt werden. - Durch das vorgeschlagene Erhitzen des Substrats auf eine Temperatur beispielsweise im Bereich von 50°C bis 100°C wird darin enthaltenes Ammonium in Form von Ammoniak entfernt. Damit kann die erste Ammonium-Stickstoffkonzentration auf einfache Weise an die jeweiligen Anforderungen angepasst werden. Die jeweiligen Anforderungen werden beeinflusst durch die Menge des Gärsubstrats, die zweite Ammonium-Stickstoffkonzentration, eine Menge eines weiteren Substrats sowie die Beschaffenheit des weiteren Substrats.

Üblicherweise ist eine dritte Ammonium-Stickstoffkonzentration des weiteren Substrats kleiner als die erste Ammonium-Stickstoffkonzentration. Infolge dessen kann auch durch die Beschaffenheit des weiteren Substrats sowie dessen Anteil im Gärsubstrat die zweite Ammonium-Stickstoffkonzentration erniedrigt werden. Das weitere Substrat kann im Wesentlichen aus nachwachsenden pflanzlichen Rohstoffen oder biogenen Reststoffen gebildet sein. Da das erfindungsgemäße Verfahren insbesondere auf eine effiziente Entsorgung hoch stickstoffhaltiger Substrate, insbesondere Geflügelkot, gerichtet ist, wird die zweite Ammonium-Stickstoffkonzentration im Gärsubstrat üblicherweise zunächst durch Zusatz von Prozesswasser erniedrigt. Sofern damit der vorgegeben Bereich der zweiten Ammonium-Stickstoffkonzentration nicht erreicht wird, kann eine weitere Erniedrigung durch Erhitzen des Substrats und/oder durch Mischen des Substrats mit dem weiteren Substrat erreicht werden.

Nach einer weiteren Ausgestaltung der Erfindung ist vorgesehen, dass zur Herstellung des Gärrests aus dem Gärsubstrat sich absetzendes Sediment mittels eines Haspelrührwerks im vergorenen Gärsubstrat suspendiert wird. Ein solchermaßen suspendierter Gärrest kann mittels einer Pumpe aus einem Gärreaktor abgeführt werden. Das ermöglicht eine besonders einfache Prozessführung.

Nach einem weiteren Ausgestaltungsmerkmal werden in einem weiteren Verfahrensschritt im Gärrest enthaltene Feststoffe mittels Mikrofiltration, vorzugsweise Ultrafiltration, abgetrennt. Die so hergestellten Pellets bilden einen wertvollen rieselfähigen Trockendünger.

Nach einer weiteren vorteilhaften Ausgestaltung handelt es sich bei dem Umkehrosmoseverfahren um ein mehrstufiges Verfahren, bei dem zweckmäßigerweise eine erste Umkehrosmose und nachfolgend eine zweite Umkehrosmose durchgeführt wird.

Vorteilhafterweise kann ein Teil einer nach dem Abtrennen der Feststoffe aus dem Gärrest verbleibenden Restflüssigkeit abgezogen und als Flüssigdünger oder zu dessen Herstellung verwendet werden. Die Restflüssigkeit wird nachfolgend vorteilhafterweise mittels des Umkehrosmoseverfahrens weiter gereinigt. Dabei fällt einerseits gereinigtes Prozesswasser an, welches zum Einstellen der zweiten Ammonium-Stickstoffkonzentration verwendet wird. Ferner fällt dabei eine weitere Restflüssigkeit an, welche wiederum als Flüssigdünger oder zu dessen Herstellung verwendet werden kann.

Falls gemäß der vorgenannten Ausgestaltung dem Verfahren Restflüssigkeit und/oder weitere Restflüssigkeit entzogen wird, wird zweckmäßigerweise ein etwa dem Volumen der abgezogenen Restflüssigkeit und/oder weiteren Restflüssigkeit entsprechendes Volumen an Flüssigkeit dem Gärsubstrat zugesetzt. Dabei kann als Flüssigkeit im Substrat oder im weiteren Substrat enthaltenes Wasser verwendet werden. Sofern dieses Wasser nicht ausreicht, kann ein ggf. verbleibendes Differenzvolumen durch die Zugabe weiterer Flüssigkeit, vorzugsweise Frischwasser, Restwasser oder Abwasser, ausgeglichen werden. Beispielsweise können als Restwasser bei der Fruchtsaftherstellung abfallende Flüssigkeiten oder dgl. zum Ausgleich des Differenzvolumens verwendet werden.

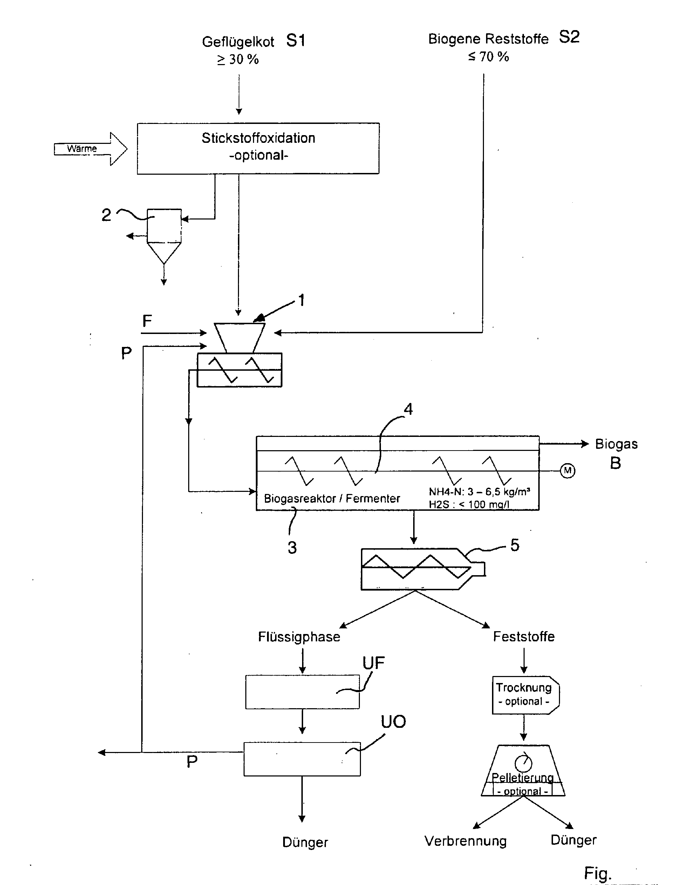

Nachfolgend wird anhand der Zeichnung ein Ausführungsbeispiel der Erfindung näher erläutert.

Die einzige Zeichnung zeigt schematisch einen Verfahrensablauf. Als Substrat S1 wird Geflügelkot mit einer ersten Ammonium-Stickstoffkonzentration von mehr als 5,0 kg NH₄-N/m³, vorzugsweise mehr als 10,0 kg NH₄-N/m³, verwendet. Als weiteres Substrat S2 werden biogene Reststoffe, beispielsweise Mais oder dgl., verwendet. Deren zweite Ammonium-Stickstoffkonzentration ist kleiner als die erste Ammonium-Stickstoffkonzentration. Sie beträgt üblicherweise weniger als 3,0 kg NH₄-N/m³. Das erste Substrat S1 und das weitere Substrat S2 werden in einen Mischer 1 gegeben. Dort wird eine Mischung hergestellt, welche zumindest 30 Gew.-% an erstem Substrat S1 und höchstens 70 Gew.-% an zweitem Substrat S2 enthält. Die Mischung wird im Mischer 1 intensiv gemischt und durch Zugabe von Frischwasser und/oder Prozesswasser und/oder Restflüssigkeit homogenisiert. Dabei entsteht eine cremige, fließfähige und damit auch pumpfähige Masse mit einem Gehalt an Trockensubstanz (TS) im Bereich von 10 bis 25 Gew.-%, vorzugsweise 15 bis 25 Gew.-%. Unter dem Begriff einer "homogenen Mischung" wird im Sinne der vorliegenden Erfindung eine Mischung verstanden, in welcher der Geflügelkot im Wesentlichen vollständig dispergiert ist. D.h. der Geflügelkot liegt in der Mischung zu einem Anteil von mehr als 90 % in einer mittleren Korngröße von weniger als 5 mm vor.

Falls erforderlich, kann das Substrat S1 vor der Zugabe zum Mischer 1 zur Erniedrigung der ersten Ammonium-Stickstoffkonzentration erhitzt werden. Dabei kann zum Erhitzen des Substrats S1 Wärme verwendet werden, welche beispielsweise aus einem mit Biogas betriebenen Blockheizkraftwerk ausgekoppelt wird. Infolge der Wärmebehandlung freigesetzter Ammoniak wird zur Neutralisation einer Neutralisiervorrichtung 2 zugeführt. Dabei kann es sich um einen Säurewäscher, beispielsweise um einen Schwefelsäurewäscher, handeln.

Zur Herstellung eines Gärsubstrats wird der Mischung Prozesswasser P und, falls erforderlich, zusätzlich Frischwasser F zugesetzt. Nach dem Einstellen einer geeigneten Konsistenz wird das so hergestellte Gärsubstrat in einem Reaktor 3 überführt. Dabei wird eine Ammonium-Stickstoffkonzentration im Reaktor 3, vorzugsweise kontinuierlich, bestimmt und geregelt, so dass eine Minimalkonzentration von 3,0 kg NH₄-N/m³ nicht unter- und eine Maximalkonzentration von 5,5 kg NH₄-N/m³ nicht überschritten wird. Zur Einhaltung des vorgenannten Konzentrationsbereichs wird Gärsubstrat einer geeigneten Ammonium-Stickstoffkonzentration zugeführt. Die H₂S-Konzentration wird im Reaktor 3 zweckmäßigerweise so eingestellt, dass sie einen Wert von 100 mg/l nicht übersteigt.

Zum Suspendieren von Feststoffen ist im Reaktor 3 ein Haspelrührwerk 4 vorgesehen. Mit dem Bezugszeichen B ist Biogas bezeichnet, welches beim Vergären des Gärsubstrats gebildet wird und aus dem Reaktor 3 zur Erzeugung von Energie abgeführt wird.

Sobald das Gärsubstrat im Wesentlichen vergoren ist, wird es mittels des Haspelrührwerks 4 suspendiert und in eine nachgeschaltete Flüssig-/Festphasentrennvorrichtung 5, z. B. einen Dekanter, gepumpt. Dort wird das im Wesentlichen vergorene Gärsubstrat in eine feste Phase und eine flüssige Phase getrennt.

Die flüssige Phase wird mittels Ultrafiltration UF gereinigt und anschließend einer, vorzugsweise zweistufigen, Umkehrosmose UO unterzogen. Nach Durchführung einer ersten Umkehrosmosestufe fällt eine flüssige Phase an, die aus dem Prozess ausgekoppelt und als Flüssigdünger verwendet werden kann. Nach Durchführung einer zweiten Umkehrosmosestufe fällt Prozesswasser P an, welches dem Mischer 1 zur Herstellung des Gärsubstrats zugeführt wird. Überschüssiges Prozesswasser P kann nötigenfalls dem Prozess entzogen werden.

Die aus der Flüssig-/Festphasentrennvorrichtung 5 abgezogenen Feststoffe können getrocknet und nachfolgend pelletiert werden. Sie können entweder als Dünger verwendet, kompostiert oder auch verbrannt werden.

### Bezugszeichenliste

- 1: Mischer
- 2: Neutralisiervorrichtung
- 3: Reaktor
- 4: Haspelrührwerk
- 5: Flüssig-/Festphasentrennvorrichtung

- B: Biogas
- F: Frischwasser
- P: Prozesswasser
- S1: Substrat
- S2: weiteres Substrat
- UF: Ultrafiltration
- UO: Umkehrosmose

## Patentansprüche

1. Verfahren zur Erzeugung von Biogas (B) aus einem Substrat (S1) mit einer ersten Ammonium-Stickstoffkonzentration von mehr als 5,0 kg NH₄-N/m³ mit folgenden Schritten:
Herstellen eines eine Mischung aus dem Substrat (S1) und weiterem Substrat (S2) enthaltenden Gärsubstrats,
Bestimmen einer zweiten Ammonium-Stickstoffkonzentration des Gärsubstrats und
Einstellen der zweiten Ammonium-Stickstoffkonzentration im Gärsubstrat derart, dass ein Minimalwert von 3,0 kg NH₄-N/m³ nicht unterschritten und ein Maximalwert von 5,5 kg NH₄-N/m³ nicht überschritten wird, wobei dem Gärsubstrat
(i) zum Erniedrigen der zweiten Ammonium-Stickstoffkon- zentration einem vergorenen Gärrest entzogenes und mittels eines Umkehrosmoseverfahrens gereinigtes Pro- zesswasser (P) und
(ii) zum Erhöhen der zweiten Ammonium-Stickstoffkonzen- tration dem Gärsubstrat Substrat (S1) zugesetzt wird.

2. Verfahren nach Anspruch 1, wobei zur Bestimmung der zweiten Ammonium-Stickstoffkonzentration der Ammoniumgehalt oder der Ammoniakgehalt im Gärsubstrat gemessen wird.

3. verfahren nach einem der vorhergehenden Ansprüche, wobei die erste Ammonium-Stickstoffkonzentration im Substrat (S1) bestimmt wird.

4. Verfahren nach einem der vorhergehenden Ansprüche, wobei zur Bestimmung der ersten Ammonium-Stickstoffkonzentration der Ammoniumgehalt oder der Ammoniakgehalt im Substrat (S1) gemessen wird.

5. Verfahren nach einem der vorhergehenden Ansprüche, wobei die erste Ammonium-Stickstoffkonzentration durch Erhitzen des Substrats (S1) vor der Zugabe zum Gärsubstrat auf einem vorgegebenen Wert verringert wird.

6. Verfahren nach einem der vorhergehenden Ansprüche, wobei der vorgegebene Wert in Abhängigkeit der zweiten Ammonium-stickstoffkonzentration sowie der Menge des Gärsubstrats und der Menge des Substrats (S1) ermittelt wird.

7. verfahren nach einem der vorhergehenden Ansprüche, wobei zum Erhitzen des Substrats (S1) durch Verbrennen von Biogas (B) erzeugtes Abgas verwendet wird.

8. Verfahren nach einem der vorhergehenden Ansprüche, wobei eine dritte Ammonium-Stickstoffkonzentration des weiteren Substrats (S2) kleiner als die erste Ammonium-Stickstoffkonzentration ist.

9. verfahren nach einem der vorhergehenden Ansprüche, wobei das Substrat (S1) im Wesentlichen aus Geflügelkot gebildet ist.

10. Verfahren nach einem der vorhergehenden Ansprüche, wobei das weitere Substrat (S2) im wesentlichen aus nachwachsenden pflanzlichen Rohstoffen oder biogenen Reststoffen gebildet ist.

11. Verfahren nach einem der vorhergehenden Ansprüche, wobei zur Herstellung des Gärrests aus dem Gärsubstrat sich absetzendes Sediment mittels eines Haspelrührwerks (4) im vergorenen Gärsubstrat suspendiert wird.

12. Verfahren nach einem der vorhergehenden Ansprüche, wobei der Gärrest mittels einer Pumpe aus einem Gärreaktor (3) abgeführt wird.

13. Verfähren nach einem der vorhergehenden Ansprüche, wobei im Gärrest enthaltene Feststoffe mittels Mikrofiltration, vorzugsweise mittels Ultrafiltration (UF), abgetrennt werden.

14. Verfahren nach einem der vorhergehenden Ansprüche, wobei die abgetrennten Feststoffe kompostiert oder pelletiert werden.

15. Verfahren nach einem der vorhergehenden Ansprüche, wobei beim Umkehrosmoseverfahren eine erste Umkehrosmose und nachfolgend eine zweite Umkehrosmose durchgeführt wird.

16. Verfahren nach einem der vorhergehenden Ansprüche, wobei ein Teil einer nach dem Abtrennen der Feststoffe aus dem Gärrest verbleibenden Restflüssigkeit abgezogen und als Flüssigdünger oder zu dessen Herstellung verwendet wird.

17. Verfahren nach einem der vorhergehenden Ansprüche, wobei die Restflüssigkeit mittels des Umkehrosmoseverfahrens gereinigt und eine dabei gebildete weitere Restflüssigkeit als Flüssigdünger oder zu dessen Herstellung verwendet wird.

18. Verfahren nach einem der vorhergehenden Ansprüche, wobei ein etwa dem Volumen der abgezogenen Restflüssigkeit und/oder weiteren Restflüssigkeit entsprechendes Volumen an Flüssigkeit (F, P) dem Gärsubstrat zugesetzt wird.

19. Verfahren nach einem der vorhergehenden Ansprüche, wobei als Flüssigkeit im Substrat (S1) oder im weiteren Substrat (S2) enthaltenes Wasser verwendet wird.

20. Verfahren nach einem der vorhergehenden Ansprüche, wobei ein ggf. verbleibendes Differenzvolumen durch die Zugabe weiterer Flüssigkeit ausgeglichen wird.

21. Verfahren nach Anspruch 20, wobei als weitere Flüssigkeit Frischwasser (F), Restwasser oder Abwasser verwendet wird.

## Claims

1. Process for the production of biogas (B) from a substrate (S1) having a first ammonium nitrogen concentration of more than 5.0 kg NH₄-N/m³ with the following steps:
Making a fermentation substrate containing a mixture from the substrate (S1) and a further substrate (S2),
Determining a second ammonium nitrogen concentration of the fermentation substrate and
Setting the second ammonium nitrogen concentration in the fermentation substrate in such a manner that a minimum value of 3.0 kg NH₄-N/m³ is not passed below and a maximum value of 5.5 kg NH₄-N/m³ is not exceeded, wherein
(i) process water (P) taken from a fermented fermentation remainder and purified via a reverse osmosis procedure is added to the fermentation substrate to lower the second ammonium nitrogen concentration and
(ii) substrate (S1) is added to the fermentation substrate to increase the second ammonium nitrogen concentration.

2. Process as defined in claim 1, wherein, to determine the second ammonium nitrogen concentration, the ammonium content or the ammonia content in the fermentation substrate is measured.

3. Process as defined in one of the preceding claims, wherein the first ammonium nitrogen concentration is determined in the substrate (S1).

4. Process as defined in one of the preceding claims, wherein, to determine the first ammonium nitrogen concentration, the ammonium content or the ammonia content in the substrate (S1) is measured.

5. Process as defined in one of the preceding claims, wherein the first ammonium nitrogen concentration is reduced to a specified value by heating the substrate (S1) before adding it to the fermentation substrate.

6. Process as defined in one of the preceding claims, wherein the specified value is determined depending on the second ammonium nitrogen concentration as well as the amount of the fermentation substrate and the amount of the substrate (S1).

7. Process as defined in one of the preceding claims, wherein exhaust gas generated by burning biogas (B) is used to heat the substrate (S1).

8. Process as defined in one of the preceding claims, wherein a third ammonium nitrogen concentration of the further substrate (S2) is less than the first ammonium nitrogen concentration.

9. Process as defined in one of the preceding claims, wherein the substrate (S1) is essentially created from chicken feces.

10. Process as defined in one of the preceding claims, wherein the further substrate (S2) is essentially created from renewable plant raw materials or biogenic remnants.

11. Process as defined in one of the preceding claims, wherein, to make the fermentation remainder, sediment settling from the fermentation substrate is suspended in the fermented fermentation substrate with a reel agitator (4).

12. Process as defined in one of the preceding claims, wherein the fermentation remainder is led out of the fermentation reactor (3) with a pump.

13. Process as defined in one of the preceding claims, wherein the solid materials contained in the fermentation remainder are separated via microfiltration, preferably via ultra filtration (UF).

14. Process as defined in one of the preceding claims, wherein the separated solid materials are composted or pelletized.

15. Process as defined in one of the preceding claims, wherein, during the reverse osmosis procedure, a first reverse osmosis and afterwards a second reverse osmosis is performed.

16. Process as defined in one of the preceding claims, wherein a part of a remainder liquid remaining after separating the solid materials from the fermentation remainder is led off and used as liquid fertilizer or for the making of same.

17. Process as defined in one of the preceding claims, wherein the remainder liquid is purified via the reverse osmosis procedure and a further remainder liquid created by this is used as liquid fertilizer or for the making of same.

18. Process as defined in one of the preceding claims, wherein a volume of liquid (F, P) corresponding approximately to the volume of the led off remainder liquid and/or further remainder liquid is added to the fermentation substrate.

19. Process as defined in one of the preceding claims, wherein water contained in the substrate (S1) or in the further substrate (S2) is used as liquid.

20. Process as defined in one of the preceding claims, wherein any eventually remaining difference volume is compensated for by adding further liquid.

21. Process as defined in claim 20, wherein fresh water (F), remainder water or waste water is used as further liquid.

## Revendications

1. Procédé de production de biogaz (B) à partir d'un substrat (S1) présentant une première concentration en azote d'ammonium supérieure à 5,0 kg NH₄-N/m³ selon les étapes suivantes:
production d'un substrat de fermentation contenant un mélange constitué du substrat (S1) et d'un autre substrat (S2),
détermination d'une seconde concentration en azote d'ammonium du substrat de fermentation et
réglage de la seconde concentration en azote d'ammonium dans le substrat de fermentation de façon telle qu'elle ne soit pas inférieure à une valeur minimale de 3,0 kg NH₄-N/m³ et pas supérieure à une valeur maximale de 5,5 kg NH₄-N/m³, où
(i) de l'eau de process (P) extraite d'un résidu de fermentation fermenté puis purifiée au moyen d'un procédé d'osmose inverse est ajoutée au substrat de fermentation afin de réduire la seconde concentra- tion en azote d'ammonium et
(ii) du substrat (S1) est ajouté au substrat de fermen- tation afin d'augmenter la seconde concentration en azote d'ammonium.

2. Procédé selon la revendication 1, selon lequel la teneur en ammonium ou la teneur en ammoniac dans le substrat de fermentation est mesurée pour la détermination de la seconde concentration en azote d'ammonium.

3. Procédé selon l'une des revendications précédentes, selon lequel la première concentration en azote d'ammonium dans le substrat (S1) est déterminée.

4. Procédé selon l'une des revendications précédentes, selon lequel la teneur en ammonium ou la teneur en ammoniac dans le substrat (S1) est mesurée pour la détermination de la première concentration en azote d'ammonium.

5. Procédé selon l'une des revendications précédentes, selon lequel la première concentration en azote d'ammonium est réduite à une valeur prédéfinie en chauffant le substrat (S1) avant de l'ajouter au substrat de fermentation.

6. Procédé selon l'une des revendications précédentes, selon lequel la valeur prédéfinie est calculée en fonction de la seconde concentration en azote d'ammonium ainsi que de la quantité du substrat de fermentation et de la quantité du substrat (S1).

7. Procédé selon l'une des revendications précédentes, selon lequel du gaz d'échappement dégagé par la combustion de biogaz (B) est utilisé pour chauffer le substrat (S1).

8. Procédé selon l'une des revendications précédentes, selon lequel une troisième concentration en azote d'ammonium de l'autre substrat (S2) est inférieure à la première concentration en azote d'ammonium.

9. Procédé selon l'une des revendications précédentes, selon lequel le substrat (S1) est constitué essentiellement de fientes de volailles.

10. Procédé selon l'une des revendications précédentes, selon lequel l'autre substrat (S2) est constitué essentiellement de matières premières végétales renouvelables ou de résidus biogènes.

11. Procédé selon l'une des revendications précédentes, selon lequel du sédiment se déposant à partir du substrat de fermentation est mis en suspension dans le substrat de fermentation fermenté au moyen d'un agitateur à palettes (4) pour la production du résidu de fermentation.

12. Procédé selon l'une des revendications précédentes, selon lequel le résidu de fermentation est évacué d'un réacteur de fermentation (3) au moyen d'une pompe.

13. Procédé selon l'une des revendications précédentes, selon lequel des matières solides contenues dans le résidu de fermentation sont séparées par microfiltration, de préférence par ultrafiltration (UF).

14. Procédé selon l'une des revendications précédentes, selon lequel les matières solides séparées sont compostées ou pelletisées.

15. Procédé selon l'une des revendications précédentes, selon lequel une première osmose inverse et successivement une seconde osmose inverse sont effectuées au cours du procédé d'osmose inverse.

16. Procédé selon l'une des revendications précédentes, selon lequel une partie d'un liquide résiduel restant du résidu de fermentation après la séparation des matières solides est retirée et utilisée comme engrais liquide ou pour sa production.

17. Procédé selon l'une des revendications précédentes, selon lequel le liquide résiduel est purifié au moyen du procédé d'osmose inverse et un autre liquide résiduel en résultant est utilisé comme engrais liquide ou pour sa production.

18. Procédé selon l'une des revendications précédentes, selon lequel un volume de liquide (F, P) correspondant approximativement au volume du liquide résiduel retiré et/ou de l'autre liquide résiduel est ajouté au substrat de fermentation.

19. Procédé selon l'une des revendications précédentes, selon lequel de l'eau contenue dans le substrat (S1) ou dans l'autre substrat (S2) est utilisée comme liquide.

20. Procédé selon l'une des revendications précédentes, selon lequel un éventuel volume différentiel restant est compensé par l'addition d'un autre liquide.

21. Procédé selon la revendication 20, selon lequel de l'eau fraîche (F), de l'eau résiduelle ou de l'eau usée est utilisée comme autre liquide.
